# EUROPEAN PATENT SPECIFICATION

(11) **EP 0 753 292 B1**
(45) Date of publication and mention of the grant of the patent: **09.04.2003**
(21) Application number: 96304833.5
(22) Date of filing: 01.07.1996
(51) Int. Cl.: A61F 13/15

(54) **Disposable absorbent undergarment of pants type**
Absorbierendes, höschenartiges Wegwerfunterkleidungstück
Sous-vêtement absorbant jetable du type couche-culotte

(30) Priority: 14.07.1995 JP 17868195
(43) Date of publication of application: 15.01.1997
(73) Proprietor: UNI-CHARM CORPORATION, Kawanoe-shi Ehime-ken (JP)
(72) Inventor: Otsubo, Toshifumi, Kawanoe-shi, Ehime-ken (JP); Soga, Hiroyuki, Mitoyo-gun, Kagawa-ken (JP)
(74) Representative: Murgatroyd, Susan Elizabeth

(56) References cited:
- EP-A- 0 183 668
- EP-A- 0 249 405
- EP-A- 0 321 732
- EP-A- 0 641 552
- WO-A-93/17648
- WO-A-96/11657
- US-A- 4 685 915

## Description

The present invention relates to disposable absorbent undergarments of pants type such as disposable diapers of pants type for babies, incontinent pants, training pants for babies and the like.

In conventional absorbent undergarments of pants type, a waist-opening and a pair of leg-openings are usually provided with respective elastic members and thereby the respective opening edges are provided with a desired stretchability in their circumferential direction. Additionally, the elastic elements associated with the waist-opening are usually arranged over a considerably large region defined between an edge of the waist-opening and a crotch region extending between the waist-opening and leg-openings so as to exert a pressure upon a location of the undergarment overlying a liquid-absorbent core from the outside and thereby to bring an inner side of this location tightly against the wearer's body. Such undergarment is disclosed, for example, in Japanese Laid-Open Patent Application Nos. Hei4-166150 and Hei4-289201.

However, attempts to bring the desired location of the undergarment overlying the liquid-absorbent core tightly against the wearer's body and thereby to avoid leakage of body fluids would disadvantageously result in causing the elastic elements to compress the wearer's waist over a wide range and often makes the undergarment less comfortable to wear.

EP-A-641552, WO-A-9317648, WO-A-9611657 and EP-A-0321732 all disclose disposable diapers having first elastic elements around the waist and leg openings. Additionally, EP-A-641552, WO-A-9317648 and WO-A-9611657 have second elastic elements disposed between the edge of the core in each of the front and rear regions of the diaper and the crotch region. In EP-A-641 552 and WO-A-9317648, the second elastic elements are equally spaced from each other and the first elastic elements around the waist opening have a greater stress than the second elastic elements. In WO-A-9641657 the spacing of the second elastic elements becomes narrower towards the waist opening and the elongation of the second elastic elements increases towards the waist opening. EP-A-183668 discloses a disposable diaper or sanitary napkin having first elastic elements around the leg openings and a network of second elastic elements to give a three-dimensional shape well adapted to the wearer's body.

Surprisingly, it has been found that leakage of body fluids can be effectively avoided by bringing only a local area overlying the longitudinally opposite edges of the core, rather than the entire location overlying the core, closely against the wearer's body and thus reduction in the comfort of the undergarment can be alleviated.

Accordingly, a principal object of the invention is the provision of a disposable absorbent undergarment of pants type in which elastic elements disposed in the front and/or rear regions of the undergarment are arranged so that the fitting of said region to the wearer's skin may be at least partially improved.

According to the invention, there is provided a disposable absorbent undergarment of pants type as claimed in claim 1.

With the disposable absorbent undergarment constructed as described above, the location of the undergarment overlying the area defined by the front and/or rear edge of the core and adjacent thereto fits well to the wearer's body without exerting a relatively high pressure, because the elastic elements associated with the front and/or rear region are arranged in this area with a relatively high density, but with a relatively lower elongation stress

The invention will be described by way of example with reference to the accompanying drawings, in which:-
Fig. 1 is a perspective view showing a diaper of pants type according to an embodiment of the invention as partially broken away; and
Fig. 2 is a sectional view taken along a line II-II in Fig. 1.

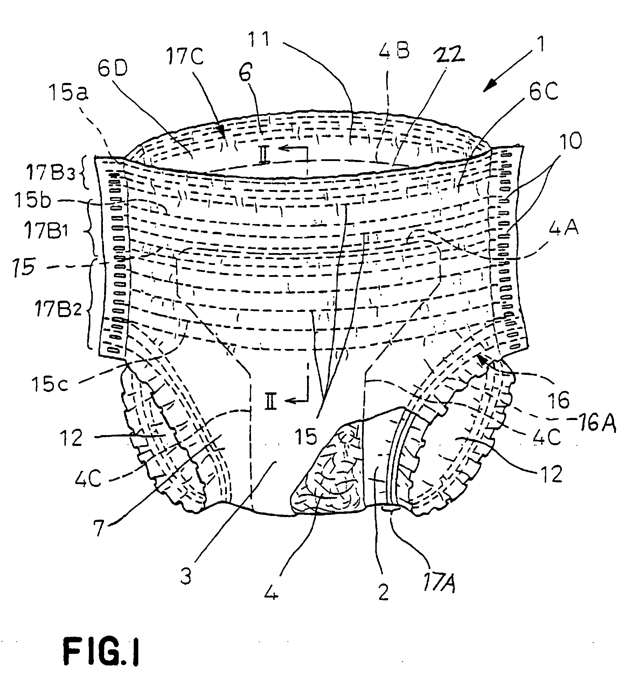

Referring to Fig. 1, a diaper comprises a liquid-permeable topsheet 2, a liquid-impermeable backsheet 3 and a liquid-absorbent core 4 disposed between these two sheets 2, 3 and further comprises a front region 5, a rear region 6 and a crotch region 7 extending between these two regions 5, 6 as viewed longitudinally of the diaper 1. The top-and backsheets 2, 3 are bonded together at their portions extending outward beyond a peripheral edge of the core 4 and the front and rear regions 5, 6 have their inner surfaces opposed to each other bonded together along their transversely opposite edges at intermittently provided adhesive spots 10 so as to define a waist-opening 11 and a pair of leg-openings 12 and thereby to form the diaper 1 as a whole in pants (or briefs)-shape. In the diaper 1, the core 4 longitudinally extends over the crotch region 7 into the front and rear regions 5, 6, and has a front edge 4A, a rear edge 4B and transversely opposite edges 4C. The diaper 1 is provided around at least the front region 5 with a first elastic member 15 and around each leg-opening with a second elastic member 16. The first and second elastic members 15, 16 are secured in extended conditions to an inner surface of at least one of the top- and backsheets 2, 3 so that a number of gathers may be formed around the waist and the legs as these elastic members 15, 16 contract.

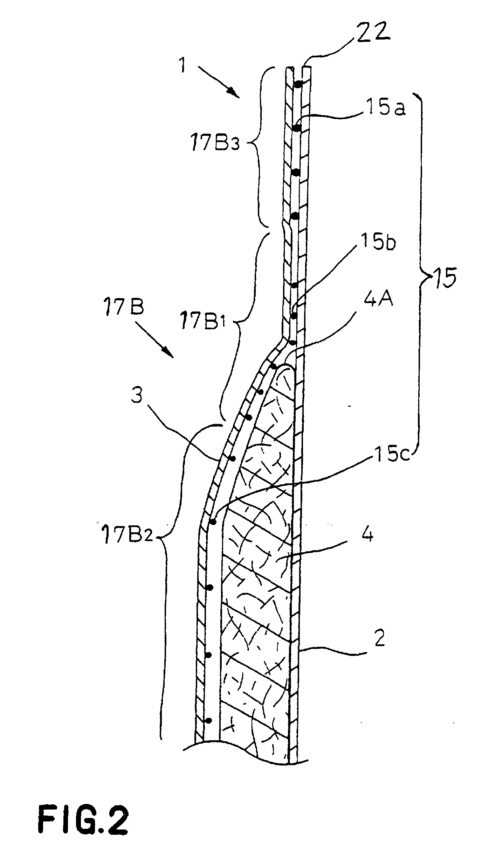

Referring to Fig. 2, the core 4 has a thickness which is substantially uniform over the front region 5, but over an area of at least 10mm deep adjacent the front edge 4A, progressively reduced toward the front edge 4A. Such locally reduced thickness may be obtained either by reducing the core material progressively toward the front edge 4A or by compressing the core material at this area. The portions of the top- and backsheets 2, 3 extending outward beyond the front edge 4A are bonded together by means of hot melt adhesive (not shown) to form a waist-opening edge 22. The rear region 6 is also constructed in a manner substantially similar to that as shown by Fig. 2.

Referring again to Figs. 1 and 2, the first elastic member 15 comprises a group 17B3 of elastic elements 15a extending along the opening edge 22, a group 17B₁ of elastic elements 15b extending adjacent the front edge 4A of the core 4 and a group 17B₂ of elastic elements 15c extending at a level lower than the group 17B₁ of elastic elements 15b. Each elastic element 15a of group 17B₃ is 0.5 to 3mm wide, 0.05 to 1.5mm thick and the number of the elastic elements 15a distributed over a depth or vertical width of 10mm from the opening edge 22 is less than two point three(2.3). Each elastic element 15b of the group 17B₁ is 0.1 to 1.5mm wide, 0.05 to 1.5mm thick and the number of the elastic elements 15b distributed over respective depths or vertical widths of 10mm above and below the front edge 4A is two point five (2.5). Each elastic element 15c of the group 17B₂ is 0.1 to 5mm wide, 0.05 to 1.5mm thick and the number of the elastic elements 15c distributed over a depth or vertical width of 10mm is two(2.0). The elastic elements 15b, 15c of the groups 17B₁ and 17B₂ intersecting the core 4 are bonded to the backsheet 3 so that they may be distributed on an outer surface of the core 4. The elastic elements 15a, 15b, 15c of the groups 17B₃, 17B₁ and 17B₂ are secured to the backsheet 3 with a 50% elongation stress per 150mm of 5 to 50g and an elongation ratio of 1.1 to 2.5. The second elastic member 16 comprises a group 17A of elastic elements 16a. The elongation stress of each of the elastic elements 15b of the group 17B₁ is lower than that of each of the elastic elements 15a, 15c of the groups 17B₃, 17B₂.

With the diaper 1 constructed as described above, the core 4 closely fits the wearer's skin at the front edge 4A and an area adjacent thereto, since the elastic elements 15b of the group 17B₁ are densely arranged above and below the front edge 4A of the core 4. Consequently, even if a portion of discharged body fluids flows on an upper surface of the topsheet 2 without being absorbed by the core 4, such flow will be unable to pass through the area adjacent the front edge 4A and will be reliably prevented from leaking outward. In the diaper 1, the elastic elements 15a of the group 17B₃ transversely extend across the front and rear regions 5, 6 so that they are continuous from the front region 5 to the rear region 6. Furthermore, an area defined by the group 17B₁ including the elastic elements 15b will not exert a greater pressure against the center zone of the wearer's stomach and will therefore not reduce the comfort of the undergarment, compared with the groups 17B₃, 17B₂ including the elastic elements 15a, 15c, since the elongation stress of each of the elastic elements 15b of the group 17B₁ is lower than that of each of the elastic elements 15a, 15c of the groups 17B₃, 17B₂. While the elastic elements 15b, 15c of the groups 17B₁, 17B₂ are preferably arranged both on the front region 5 and on the rear region 6, it is also possible without departing from the scope of the invention to arrange them on either one of these front and rear regions, for example, depending on the wearer's particular body form.

The elastic elements 15b of the group 17B may be replaced by one or more relatively wide ribbon-like elastic member(s) to improve the fitting of the core at its front edge 4A and area therearound to the wearer's skin. However, such replacement would be disadvantageous in that a desired airpermeation between the interior and exterior of the diaper 1 will be significantly impeded.

In order to implement the invention, a nonwoven fabric or perforated plastic film may be used for the topsheet 2 and a plastic film such as polyethylene may be used for the backsheet 3. Fluff pulp alone or a mixture of fluff pulp and superabsorptive polymer powder may be molded by any well known technique into a shape required for the core 4. A material having a rubber-like elasticity such as rubber or plastic elastomer may be used for the first and second elastic members 15, 16.

With the undergarment of the invention, the undergarment reliably fits the wearer's body at the area adjacent the front edge of the core and can prevent body fluids from leaking beyond this edge without reducing its comfort to wear. Furthermore, the arrangement such that the thickness of the core adjacent the front and/or rear edge(s) thereof is progressively reduced toward the edge(s) is effective to reduce a difference in level generated between the core and the wearer's skin and thereby to improve the appearance of the undergarment whilst being worn. Even if such progressive decrease in the core thickness is obtained by compressing the core material, the densely arranged elastic elements allow such progressively thinned area to fit the wearer's body.

## Claims

1. A disposable absorbent undergarment (1) of pants type comprising a liquid-permeable topsheet (2), a liquid-impermeable backsheet (3) and a liquid-absorbent core (4) disposed therebetween, said undergarment (1) having a front region (5), a rear region (6) and a crotch region (7) extending therebetween, said core (4) longitudinally extending into said front and rear regions (5,6) and having front and rear edges (4A,4B) spaced apart from each waist-opening edge (22) of said front and rear regions (5,6) respectively; a first elastic member (15) comprising a first group (17B₃) of elastic elements (15a) extending along the waist-opening edge (22) of at least said front region (5) and a second group (17B₁) of elastic elements (15b) extending adjacent at least one of said front and rear edges (4A,4B) of said core (4) both above and below said at least one edge (4A,4B); and a second elastic member (16) provided around each leg-opening (12); **characterized in that**:-
said first elastic member (15) also comprises a third group (17B₂) of elastic elements (15c) extending at a level of said front and/or rear region (5,6) lower than said second group (17B₁) of elastic elements (15b);
the density of said elastic elements (15b) of said second group (17B₁) is higher than the density of said elastic elements (15a, 15c) of said first and third groups (17B₃,17B₂); and
the elongation stress of each elastic element (15b) of said second group (17B₁) is lower than the elongation stress of each elastic element (15a, 15c) of said first and third groups (17B₃, 17B₂).

2. The undergarment (1) according to claim 1, wherein, in an area of said core (4) at least 10mm inward from the front edge (4A) of said core (4), said core (4) has a thickness progressively reduced toward said front edge (4A).

3. The undergarment (1) according to claim 1 or 2, wherein, in an area of said core (4) at least 10mm inward from the rear edge (4B) of said core (4), said core (4) has a thickness progressively reduced toward said rear edge (4B).

4. The undergarment (1) according to Claim 1, 2, or 3, wherein said second elastic elements (15b, 15c) are arranged on both said front and rear regions (5, 6).

## Patentansprüche

1. Absorbierende Wegwerfunterwäsche (1) in Höschenform, umfassend eine flüssigkeitsdurchlässige Oberschicht (2), eine flüssigkeitsundurchlässige Unterschicht (3) und einen dazwischen angeordneten, flüssigkeitsaufnehmenden Kern (4), wobei die Unterwäsche (1) einen vorderen Bereich (5), einen hinteren Bereich (6) und einen sich dazwischen erstreckenden Schrittbereich (7) umfasst, wobei sich der Kern (4) längsweise in den vorderen und den hinteren Bereich (5, 6) hinein erstreckt und vordere und hintere Ränder (A4, 4B) aufweist, die von jedem Taillenöffnungsrand (22) der vorderen und hinteren Bereiche (5, 6) jeweils beabstandet sind; ein erstes elastisches Glied (15), das eine erste Gruppe (17B₃) elastischer Elemente (15a) umfasst, die sich an dem Taillenöffnungsrand (22) entlang mindestens des vorderen Bereiches (5) erstrecken, und eine zweite Gruppe (17B₁) elastischer Elemente (15b), die sich angrenzend an mindestens einen der vorderen und hinteren Ränder (4A, 4B) des Kernes (4) sowohl oberhalb als auch unterhalb des mindestens einen Randes (4A, 4B) erstrecken; und ein zweites elastisches Glied (16), das um jede Beinöffnung (12) vorgesehen ist, **dadurch gekennzeichnet, dass**
das erste elastische Glied (15) ebenfalls eine dritte Gruppe (17B₂) elastischer Elemente (15c) umfasst, die sich in Höhe des vorderen und/oder hinteren Bereiches (5, 6) unterhalb der zweiten Gruppe (17B₁) elastischer Elemente (15b) erstrecken;
dass die Dichte der elastischen Elemente (15b) der zweiten Gruppe (17B₁) größer ist als die Dichte der elastischen Elemente (15a, 15c) der ersten und dritten Gruppe (17B₃, 17B₂); und
dass die Dehnungsspannung jedes elastischen Elementes (15b) der zweiten Gruppe (17B₁) niedriger ist als die Dehnungsspannung jedes elastischen Elementes (15a, 15c) der ersten und der dritten Gruppe (17B₃, 17B₂).

2. Die Unterwäsche (1) nach Anspruch 1, bei der in einer Zone des Kemes (4) mindestens 10 mm einwärts von dem vorderen Rand (4A) des Kernes (4) der Kem (4) eine Dicke aufweist, die zum vorderen Rand (4A) hin progressiv abnimmt.

3. Die Unterwäsche (1) nach Anspruch 1 oder 2, bei der in einer Zone des Kernes (4) mindestens 10 mm einwärts von dem hinteren Rand (4B) des Kernes (4) der Kem (4) eine Dicke aufweist, die zum hinteren Rand (4B) hin progressiv abnimmt.

4. Die Unterwäsche (1) nach Anspruch 1, 2 oder 3, bei der die zweiten elastischen Elemente (15b, 15C) sowohl in dem vorderen als auch in dem hinteren Bereich (5,6) angeordnet sind.

## Revendications

1. Sous-vêtement absorbant jetable (1) du type culotte comprenant une feuille supérieure perméable aux liquides (2), une feuille inférieure imperméable aux liquides (3) et un noyau absorbant les liquides (4) disposé entre elles, ledit sous-vêtement (1) ayant une zone avant (5), une zone arriére (6) et une zone d'entrejambe (7) s'étendant entre elles, ledit noyau (4) s'étendant longitudinalement dans lesdites zones avant et arrière (5, 6) et ayant des bords avant et arrière (4A, 4B) espacés de chaque bord d'ouverture de ceinture (22) desdites zones avant et arrière (5, 6) respectivement, un premier élément élastique (15) comprenant un premier groupe (17B₃) d'éléments élastiques (15A) s'étendant le long du bord d'ouverture de ceinture (22) d'au moins ladite zone avant (5) et un second groupe (17B₁) d'éléments élastiques (15B) s'étendant de manière adjacente à au moins un desdits bords avant et arrière (4A, 4B) dudit noyau (4) à la fois au dessus et en dessous dudit au moins un bord (4A, 4B); et un second élément élastique (16) prévu autour de chaque ouverture de jambe (12) ; **caractérisé en ce que** :
ledit premier élément élastique (15) comprend également un troisième groupe (17B₂) d'élément élastique (15C) s'étendant à un niveau de ladite zone avant et/ou arrière (5, 6) plus bas que ledit second groupe (17B₁) d'élément élastique (15B) ;
la densité desdits éléments élastiques (15B) dudit second groupe (17B₁) est supérieure à la densité desdits éléments élastiques (15A, 15C) desdits premier et troisième groupes (17B₃, 17B₂);
la contrainte d'élongation de chaque élément élastique (15B) dudit second groupe (17B₁) est inférieure à la contrainte d'élongation de chaque élément élastique (15A, 15C) desdits premier et troisième groupes (17B₃,17B₂).

2. Sous-vêtement (1) selon la revendication 1, dans lequel, dans une zone dudit noyau (4), au moins dix millimètres vers l'intérieur à partir du bord avant (4A) dudit noyau (4), ledit noyau (4) a une épaisseur qui se réduit progressivement en direction dudit bord avant (4A).

3. Sous-vêtement (1) selon la revendication 1 ou 2, dans lequel, dans une zone dudit noyau (4), à au moins 10 millimètres vers l'intérieur à partir du bord arrière (4B) dudit noyau (4), ledit noyau (4) a une épaisseur qui se réduit progressivement en direction du bord arrière (4B).

4. Sous-vêtement (1) selon la revendication 1, 2 ou 3, dans lequel lesdits seconds éléments élastiques (15B, 15C) sont arrangés à la fois sur ladite zone avant (5) et sur ladite zone arrière (6).
